(19) Europäisches Patentamt
European Patent Office
Office européen des brevets

(11) Numéro de publication: 0 131 981
A1

# (12) DEMANDE DE BREVET EUROPEEN

(21) Numéro de dépôt: 84200845.0

(22) Date de dépôt: 13.06.84

(51) Int. Cl.[4]: C 07 D 307/42
C 07 D 307/68, C 07 D 307/12

(30) Priorité: 29.06.83 FR 8310950

(43) Date de publication de la demande: 23.01.85 Bulletin 85/4

(84) Etats contractants désignés: AT BE CH DE GB IT LI LU NL SE

(71) Demandeur: AGRIFURANE S.A.
Z.I. de Boé Cidex 4412
F-47240 Bon Encontre(FR)

(72) Inventeur: Le Bigot, Yves
Mas de Roux Bragassargues
F-30260 Quissac(FR)

(72) Inventeur: Audoye, Paul
Rue Jean Moulin
F-09400 Tarascon Sur Ariege(FR)

(72) Inventeur: Delmas, Michel
23, résidence des Amandiers Deyme
F-31450 Montgiscard(FR)

(72) Inventeur: Gaset, Antoine
75, allée de Brienne
F-31000 Toulouse(FR)

(74) Mandataire: Barre, Philippe
Cabinet Barre-Gatti-Laforgue 95 rue des Amidonniers
F-31069 Toulouse Cédex(FR)

## (54) Procédé de préparation d'esters furanniques par réaction de transestérification.

(57) L'invention concerne un procédé de préparation d'un ester furannique par une réaction de transestérification d'un autre ester en présence d'un catalyseur constitué par un carbonate alcalin autre que le carbonate de lithium. Le procédé consiste à réaliser la réaction de transestérification avec un alcool furannique à une température comprise entre 0° C et 90° C. L'alcool utilisé peut être l'alcool furfurylique. Le procédé conforme à l'invention permet en particulier de préparer le furoate de furfuryle à partir du furoate d'éthyle et de l'alcool furfurylique.

EP 0 131 981 A1

Croydon Printing Company Ltd.

# PROCEDE DE PREPARATION D'ESTERS FURANNIQUES PAR REACTION DE TRANSESTERIFICATION

L'invention concerne un procédé de préparation d'un ester furannique par une réaction de transestérification à partir d'un autre ester ; elle s'applique notamment pour l'obtention d'un ester furfurylique. Elle vise des procédés du type dans lesquels la réaction de transestérification est réalisée en présence d'un catalyseur basique de transestérification, constitué par un carbonate alcalin autre que le carbonate de lithium.

On sait que les réactions de transestérification ont pour but de fabriquer un ester difficile à obtenir par les méthodes traditionnelles (synthèse), à partir d'un autre ester qui, lui, est au contraire facile à obtenir par les méthodes traditionnelles à un prix de revient modéré.

Il est décrit dans le brevet US n° 2 433 866 une réaction de transestérification en vue de la fabrication du furfurylacrylate ; cette réaction se déroule en présence de tertio butylate d'aluminium qui joue le rôle de catalyseur, et d'un inhibiteur de polymérisation ; elle conduit à un mélange d'ester et d'inhibiteur ce qui constitue son inconvénient essentiel ; en effet les inhibiteurs de polymérisation sont des produits très toxiques et les esters ainsi réalisés sont inutilisables dans de nombreux domaines (agroalimentaire, pharmaceutique, cosmétique...). En l'absence d'inhibiteur, cette réaction s'accompagne d'une réticulation spontanée conduisant à des polymères.

Par ailleurs, il est décrit dans le brevet US n° 3 996 206, le brevet FR n° 2 487 838 et d'autres publications (en particulier A. UJHIDY et al "Méthod for the preparation of fatty acid-sugar esters" HUNG J. IND. CHEM. 1973. 4 (1), 513-32) des méthodes de transestérification, agissant en phase liquide /liquide en présence de carbonate alcalin, en vue d'obtenir des surfactants (agents actifs des détergents). Ces procédés se caractérisent en ce que le réactif de la transestérification est constitué par du saccharose et que la réaction est réalisée à des températures comprises entre 110° C et 165° C, devant être en tout cas supérieures à

110° C. Ces procédés de préparation présentent un inconvénient majeur qui réside dans la dépense énergétique importante nécessaire pour porter les corps à la température requise ; dans les procédés industriels, des poids très importants de réactifs (plusieurs tonnes) sont mis en jeu et cette consommation énergétique est un des facteurs essentiels, qui obère le coût des nouveaux esters obtenus. Par ailleurs, ces réactions sont limitées à la transestérification de certains esters constitués par les triglycérides, les esters obtenus étant des esters lourds dérivés du saccharose, ayant une molécule très oxygénée.

Or, il existe de nombreux autres esters qui sont très difficiles et très chers à obtenir par des méthodes traditionnelles et qui ont des propriétés extrêmement intéressantes dans de nombreux secteurs industriels (propriétés ignifuges, aromatiques, analgésiques, insecticides, etc...).

La présente invention se propose d'indiquer une nouvelle réaction de transestérification qui puisse être mise en oeuvre à la température ambiante ou à basse température, de façon à n'exiger aucune consommation énergétique ou une consommation énergétique négligeable.

Un autre objectif de l'invention est de fournir une réaction de transestérification permettant d'obtenir de nombreux types d'esters furanniques ayant des substituants variés. En particulier, l'invention vise une réaction de transestérification particulièrement performante pour l'obtention d'esters intégrant le noyau furannique (esters furfuryliques)

A cet effet, le procédé de préparation visé par l'invention met en oeuvre une réaction de transestérification d'un ester de formule $R_1 - \overset{O}{\overset{\|}{C}} - O - R_5$, où $R_1$ et $R_5$ sont des radicaux hydrocarbonés contenant au moins un hétéroatome, en présence d'un catalyseur constitué par un carbonate alcalin autre que le carbonate de lithium ; selon la présente invention, en vue d'obtenir un ester furannique de formule

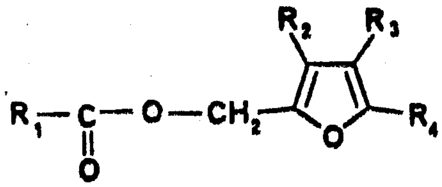 ou 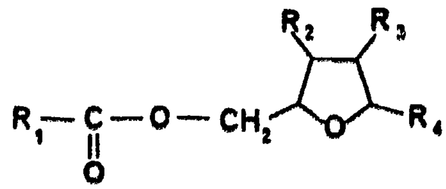

où $R_2$, $R_3$ et $R_4$ sont des hydrogènes ou des radicaux hydrocarbonés contenant au moins un hétéroatome, on réalise la réaction de transestérification de l'ester $R_1 - \overset{O}{\overset{\|}{C}} - O - R_5$ avec un alcool furannique de formule

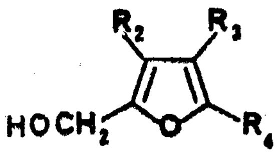 ou 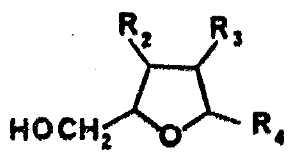

à une température comprise entre 0° C et 90° C.

En particulier, le procédé de l'invention permet de fabriquer des esters furfuryliques qui ont des propriétés connues d'agents ignifuges, aromatisants, bactéricides, en utilisant comme alcool, l'alcool furfurylique

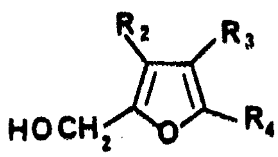

ou l'alcool tetrahydrofurfurylique

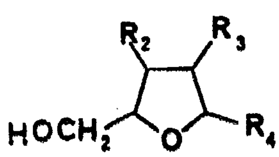

On a pu constater de façon inattendue que la combinaison :

. d'un catalyseur constitué par un carbonate alcalin (autre que le carbonate de lithium),

. d'un réactif de transestérification constitué par un alcool furannique,

. et d'une température douce comprise entre 0° C et 90° C

conduisait à une réaction en phase hétérogène, bénéficiant

d'un excellent rendement en ester, en l'absence de tout corps additionnel. La température choisie peut être la température ambiante ; dans la plupart des cas, on préfèrera une température comprise entre 40° et 60° C.

Ce résultat remarquable peut s'expliquer par les phénomènes originaux qui se développent dans cette réaction : la transestérification se déroule en phase hétérogène par transfert solide/liquide entre le catalyseur à l'état solide et les réactifs en phase liquide (alcool, ester de base, ester produit). Au contraire, dans l'art antérieur, utilisant comme catalyseur un carbonate alcalin, une proportion substantielle du catalyseur passe en phase liquide et la transestérification s'effectue essentiellement en phases liquide/liquide, ce qui exige un apport énergétique beaucoup plus important. Ces faits expliquent les idées préconçues existant dans la technique, qui ont porté l'homme du métier à croire que seules des températures élevées permettaient le déroulement de réactions de transestérification avec ce type de catalyseur.

De préférence, le catalyseur utilisé est le carbonate de potassium.

De plus, les expérimentations ont démontré que la réaction de transestérification visée par l'invention peut être mise en oeuvre sans solvant auxiliaire, l'alcool étant prévu en excès (par rapport aux proportions stoechiométriques) et jouant le rôle de solvant. Cette condition remarquable constitue en pratique un avantage essentiel car elle facilite l'extraction industrielle de l'ester obtenu, par filtration du catalyseur suivi d'une distillation pour supprimer l'excès d'alcool, lequel peut être recyclé.

A la pression atmosphérique, il est préférable de prévoir un large excès d'alcool correspondant à un rapport molaire $\frac{\text{alcool furannique}}{\text{ester de départ}}$ supérieur ou égal à 5 ; en effet, cet excès déplace l'équilibre de la réaction et conditionne un bon rendement de celle-ci.

Il est également possible de travailler à pression réduite et l'excès d'alcool peut alors être beaucoup plus faible (rapport molaire compris entre 1,5 et

3). L'abaissement de la pression permet l'extraction continue (par distillation spontanée) de l'alcool formé et déplace l'équilibre vers la formation de l'ester furannique, conditionnant ainsi un bon rendement de la réaction. En pratique, l'on pourra prévoir une pression comprise entre 10 et 50 mm Hg (1315 à 6575 Pascals).

Selon une autre condition préférentielle de mise en oeuvre du procédé de l'invention, la proportion de catalyseur mise en oeuvre est comprise entre 0,05 et 0,7 mole de catalyseur par mole d'ester $R_1 - \overset{O}{\overset{\|}{C}} - O - R_5$.

Les exemples qui suivent illustrent le procédé de l'invention dans le cas d'une mise en oeuvre à pression atmosphérique (exemples 1 à 9) et dans le cas d'une mise en oeuvre à pression réduite (exemples 10 à 15).

<u>Mode opératoire concernant les exemples 1 à 9 réalisés à pression atmosphérique</u>

Synthèse d'un ester de furfuryle par transestérification entre un ester d'alkyle et l'alcool furfurylique en grand excès en présence de carbonate de potassium.

Dans un réacteur de 100 ml, on place un excès d'alcool furfurylique, 0,02 mole d'ester d'alkyle et une quantité variable qui peut être comprise entre 1 et 10 g de carbonate alcalin. Le milieu réactionnel est agité à une température variant entre 25° C et 60° C et pour une durée correspondante indiquée dans le tableau I.

Ces conditions permettent l'obtention des esters furanniques par distillation, récupération et recyclage de l'alcool furfurylique en excès après avoir filtré la phase solide. Les esters furanniques sont ensuite obtenus purs par distillation sous vide et ont été identifiés par leurs constantes physicochimiques et leurs caractéristiques spectroscopiques (résonance magnétique du proton et du carbone 13, Infra-Rouge).

Le tableau I ci-après résume les résultats obtenus.

TABLEAU I

| Exemples | Ester furannique synthétisé | [alcool furfurylique] / [ester de départ] (rapport molaire) | Durée de la réaction (heures) | Température de la réaction | Nature du catalyseur et quantité en grammes | Rendement* (%) |
|---|---|---|---|---|---|---|
| 1 | acétate de furfuryle | 5 | 8 | 60° C | $K_2CO_3$ (8 g) | 75 |
| 2 | acétate de furfuryle | 10 | 6 | 40° C | $K_2CO_3$ (3 g) | 76 |
| 3 | acétate de furfuryle | 10 | 24 | 25° C | $K_2CO_3$ (4 g) | 80 |
| 4 | acétate de furfuryle | 8 | 4 | 60° C | $Na_2CO_3$ (4 g) | 65 |
| 5 | acétate de furfuryle | 10 | 4 | 50° C | $Rb_2CO_3$ (4 g) | 80 |
| 6 | acétate de furfuryle | 10 | 4 | 50° C | Cs $CO_3$ (4 g) | 76 |
| 7 | butyrate de furfuryle | 8 | 4 | 60° C | $K_2CO_3$ (4 g) | 78 |
| 8 | benzoate de furfuryle | 10 | 4 | 50° C | $K_2CO_3$ (4 g) | 80 |
| 9 | furoate de furfuryle | 10 | 6 | 50° C | $K_2CO_3$ (6 g) | 76 |

* La réaction est totalement sélective. Il ne se forme pas de produits secondaires. Le complément à 100 du rendement est constitué par de l'ester de départ qui n'a pas réagi.

**Mode opératoire concernant les exemples 10 à 15 réalisés à pression réduite obtenue à la trompe à eau**

Synthèse d'un ester de furfuryle par transestérification entre un ester d'alkyle et l'alcool furfurylique à pression réduite obtenue par une trompe à eau (cette technique, très facile à mettre en oeuvre à l'échelle industrielle, n'exige pas des caractéristiques mécaniques contraignantes pour l'installation et ne grève pas, par rapport aux réactions réalisées à température ambiante, les coûts de fabrication).

On place dans un réacteur de 250 ml une mole d'alcool furfurylique et une quantité d'ester d'alkyle et de carbonate alcalin variable (tableau II) et on fait un vide partiel dont la valeur est précisée dans le tableau II. Le milieu réactionnel est agité et chauffé à une température indiquée dans le tableau II pendant trois heures. L'alcool méthylique ou éthylique formé dans le cas des exemples choisis distille en continu pendant la réaction et est récupéré au moyen d'un appareil de "DEAN-STARK". En fin de réaction, le milieu réactionnel est distillé. L'alcool furfurylique en léger excès est récupéré et recyclé. Les esters furanniques sont ensuite obtenus purs par distillation sous vide et ont été identifiés par leurs constantes physicochimiques et leurs caractéristiques spectroscopiques (résonance magnétique nucléaire du proton et du carbone 13, Infra-Rouge).

Le tableau II résume les résultats obtenus.

TABLEAU II

| Exemples | Ester furannique synthétisé | [alcool furfurylique] / [ester de départ] (rapport molaire) | Température de la réaction | Pression (en mm Hg) | Nature du catalyseur et quantité en grammes | Rendement* (%) |
|---|---|---|---|---|---|---|
| 10 | acétate de furfuryle | 2 | 60° C | 50 | $K_2CO_3$ (15 g) | 90 |
| 11 | acétate de furfuryle | 1,5 | 55° C | 30 | $K_2CO_3$ (20 g) | 88 |
| 12 | acétate de furfuryle | 3 | 50° C | 25 | $Kb_2CO_3$ (10g) | 92 |
| 13 | furoate de furfuryle | 3 | 50° C | 20 | $K_2CO_3$ (20 g) | 85 |
| 14 | palmitate de furfuryle | 3 | 60° C | 50 | $K_2CO_3$ (20 g) | 94 |
| 15 | stéarate de furfuryle | 3 | 60° C | 50 | $K_2CO_3$ (20 g) | 90 |

* La réaction est totalement sélective. Il ne se forme pas de produits secondaires. Le complément à 100 du rendement est constitué par l'ester de départ qui n'a pas réagi.

Les exemples précédents ont été mis en oeuvre en utilisant l'alcool furfurylique

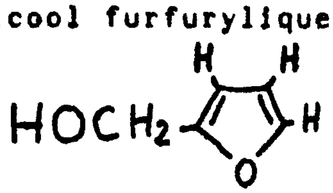

les deux exemples qui suivent illustrent l'utilisation de l'alcool tetrahydrofurfurylique

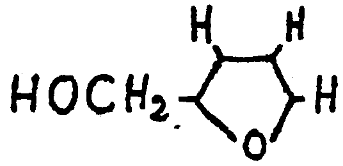

Exemple 16

Synthèse d'un ester de furfuryle par transestérification entre un ester d'alkyle et l'alcool tétrahydrofurfurylique en excès en présence de carbonate de potassium.

Dans un réacteur de 100 ml, on place 0,08 mole d'alcool furfurylique, 0,02 mole d'acétate d'éthyle et une quantité de 3 g de carbonate alcalin. Le milieu réactionnel est agité à une température de 50° C pour une durée de trois heures.

Ces conditions permettent l'obtention de l'ester furannique par distillation, récupération et recyclage de l'alcool tétrahydrofurfurylique en excès après avoir filtré la phase solide, avec un rendement de 92 % : l'ester furannique est ensuite obtenu pur par distillation sous vide et a été identifié par ses constantes physicochimiques et ses caractéristiques spectroscopiques (résonance magnétique du proton et du carbone 13, Infra-Rouge).

Exemple 17

Synthèse d'un ester de furfuryle par transestérification entre l'acétate d'éthyle et l'alcool tétrahydrofurfurylique à pression réduite obtenue par une trompe à eau.

On place dans un réacteur de 250 ml une mole d'alcool tétrahydrofurfurylique, 1,5 mole d'acétate d'éthyle et 20 g de carbonate alcalin et on fait un vide partiel égal à 200 mm Hg (2630 Pascals). Le milieu réactionnel est agité et chauffé à une température de 60° C pendant trois heures. L'alcool méthylique formé dans ce cas distille en continu pendant la réaction et est récupéré au moyen d'un appareil de "DEAN-STARK". En fin de réaction, le milieu réactionnel est distillé. L'alcool tétrahydrofurfurylique en léger excès est récupéré et recyclé. L'ester furannique est ensuite obtenu pur par distillation sous vide avec un rendement de 95 % et a été identifié par ses constantes physicochimiques et ses caractéristiques spectroscopiques (résonance magnétique nucléaire du proton et du carbone 13, Infra-Rouge).

REVENDICATIONS

1/ - Procédé de préparation d'un ester par une réaction de transestérification d'un autre ester de formule $R_1 - \overset{O}{\overset{\|}{C}} - O - R_5$, où $R_1$ et $R_5$ sont des radicaux hydrocarbonés contenant au moins un hétéroatome, en présence d'un catalyseur constitué par un carbonate alcalin autre que le carbonate de lithium, caractérisé en ce que, en vue d'obtenir un ester furannique de formule

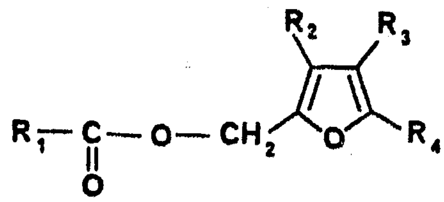

ou

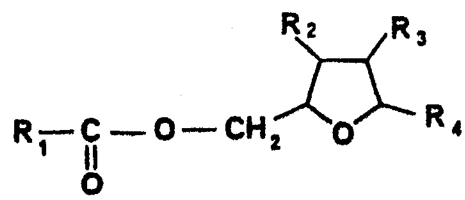

où $R_2$, $R_3$ et $R_4$ sont des hydrogènes ou des radicaux hydrocarbonés contenant au moins un hétéroatome, la réaction de transestérification de l'ester $R_1 - \overset{O}{\overset{\|}{C}} - O - R_5$ est réalisée avec un alcool furannique de formule

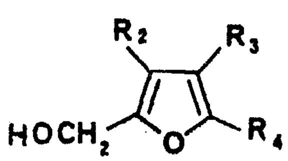

ou

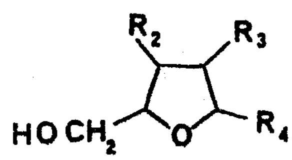

à une température comprise entre 0° C et 90° C.

2/ - Procédé de préparation d'un ester furfurylique selon la revendication 1, caractérisé en ce que l'on utilise, comme alcool, l'alcool furfurylique dans lequel les radicaux $R_2$, $R_3$ et $R_4$ sont des hydrogènes.

3/ - Procédé de préparation selon l'une des revendications 1 ou 2, caractérisé en ce que la réaction de transestérification est réalisée à température

comprise entre 40° C et 60° C.

4/ - Procédé selon l'une des revendications 2 ou 3 pour préparer le furoate de furfuryle, caractérisé en ce que l'on réalise la réaction de transestérification à partir du furoate d'éthyle et de l'alcool furfurylique.

5/ - Procédé selon l'une des revendications 1, 2, 3 ou 4, dans lequel l'on utilise comme catalyseur le carbonate de potassium.

6/ - Procédé selon l'une des revendications 1, 2, 3, 4 ou 5, caractérisé en ce que la réaction de transestérification est réalisée en présence d'un excès d'alcool qui sert de solvant.

7/ - Procédé selon la revendication 6, caractérisé en ce que la réaction de transestérification est réalisée à la pression atmosphérique en présence d'un excès d'alcool correspondant à un rapport molaire $\frac{\text{alcool furannique}}{\text{ester de départ}}$ supérieur ou égal à 5.

8/ - Procédé selon la revendication 6, caractérisé en ce que la réaction de transestérification, est réalisée à une pression comprise entre 10 et 50 mm Hg (soit entre 1315 pascals et 6575 pascals) en présence d'un léger excès d'alcool correspondant à un rapport molaire $\frac{\text{alcool furannique}}{\text{ester de départ}}$ compris entre 1,5 et 3.

9/ - Procédé selon l'une des revendications 1, 2, 3, 4, 5, 6, 7 ou 8, caractérisé en ce que la proportion de catalyseur mise en oeuvre est comprise entre 0,05 et 0,7 mole de catalyseur par mole d'ester

$$R_1 - \overset{\overset{O}{\|}}{C} - O - R_5.$$

Office européen des brevets

# RAPPORT DE RECHERCHE EUROPEENNE

0131981
Numéro de la demande

EP 84 20 0845

## DOCUMENTS CONSIDERES COMME PERTINENTS

| Catégorie | Citation du document avec indication, en cas de besoin des parties pertinentes | Revendication concernée | CLASSEMENT DE LA DEMANDE (Int. Cl. 3) |
|---|---|---|---|
| D,Y | US-A-2 433 866 (C.E. REHBERG) * en entier * | 1-9 | C 07 D 307/42<br>C 07 D 307/68<br>C 07 D 307/12 |
| D,Y | CHEMICAL ABSTRACTS, vol. 81, no. 20, 18 novembre 1974, page 173, no. 123316n, Columbus, Ohio, US; A. UJHIDY et al.: "Method for the preparation of fatty acid-sugar esters" & HUNG. J. IND. CHEM. 1973, 4(1), 513-32 | 1-9 | |
| | | | **DOMAINES TECHNIQUES RECHERCHES (Int. Cl. 3)** |
| | | | C 07 D 307/00 |

Le présent rapport de recherche a été établi pour toutes les revendications

| Lieu de la recherche | Date d'achèvement de la recherche | Examinateur |
|---|---|---|
| LA HAYE | 03-10-1984 | ALLARD M.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82